# DEMANDE DE BREVET EUROPEEN

(11) **EP 1 834 914 A1**
(43) Date de publication de la demande: **19.09.2007**
(21) Numéro de dépôt: 07103981.2
(22) Date de dépôt: 13.03.2007
(51) Int. Cl.: B65H 37/00, A61M 35/00, A61Q 19/00

(54) **Dispositif de transfert d'un produit provenant d'une bande support**

(30) Priorité: 14.03.2006 FR 0602203
(71) Demandeur: L'Oréal, 75008 Paris (FR)
(72) Inventeur: Habatjou, Jacques, 59400 Cambrai (FR)
(74) Mandataire: de Kernier, Gabriel

(57) **Abrégé**

Ce dispositif de transfert d'au moins un produit (12) disposé sur une bande support (14), est pourvu d'un boîtier (16), d'une bobine d'alimentation (32) sur laquelle est enroulée la bande support du produit à transférer, et d'une bobine de réception (34) de la bande support, les bobines d'alimentation et de réception étant montées à rotation sur le boîtier.

La bande support comprend une pluralité de produits prédécoupés.

Le dispositif comprend en outre un organe d'actionnement (20) mobile par rapport au boîtier et apte à entraîner en rotation l'une desdites bobines de manière à permettre un déroulement d'une longueur prédéterminée de la bande support.

L'invention trouve application pour le dépôt d'un patch cosmétique ou de soin sur l'épiderme.

## Description

La présente invention concerne l'application d'un timbre ou patch contenant une composition cosmétique ou de soin sur une surface d'application, en particulier sur une partie à traiter de l'épiderme d'un utilisateur ou d'une utilisatrice.

Plus particulièrement, l'invention se rapporte à un dispositif pour l'application d'un patch sur la peau d'un utilisateur ou d'une utilisatrice.

Une des applications intéressantes de l'invention concerne l'application d'un patch adhésif comprenant une composition traitante ou hydratante, ou comprenant encore une composition anti-rides.

Mais l'invention concerne, de manière générale, un dispositif pour appliquer un produit sur une surface d'application, ce produit étant prélevé à partir d'une bande support.

De tels dispositifs de transfert sont généralement utilisés dans le domaine de la papeterie, et plus particulièrement pour appliquer un film opaque blanc sur une feuille de papier en vue d'une correction d'écriture.

Ils comprennent classiquement un boîtier à l'intérieur duquel sont montées une bobine d'alimentation sur laquelle est enroulée une bande support du film à transférer, et une bobine de réception de la bande support, une fois le film transféré sur la feuille de papier.

A cet égard, les dispositifs connus comprennent un organe de transfert ou d'application en saillie par rapport au boîtier et autour duquel la bande support forme une boucle. Pour plus de détails, on pourra par exemple se référer au dispositif décrit dans le brevet européen EP-B1-1 025 030.

Ce dispositif est particulièrement adapté pour le transfert d'un film de recouvrement dans le domaine de la papeterie. I1 est par contre inadapté pour le transfert d'un patch cosmétique sur un épiderme. En effet, pour une application cosmétique, ce dispositif présente l'inconvénient de nécessiter d'exercer une pression relativement importante sur une partie du corps d'un utilisateur à traiter pour assurer la rotation de la bobine d'alimentation en vue du transfert du patch. Ainsi, lors d'un tel transfert, cette surpression à exercer peut provoquer pour l'utilisateur une appréciation négative du dispositif, voire une blessure.

Par ailleurs, dans le cas d'une bande support sur laquelle est disposée une pluralité de films ou patchs prédécoupés et espacés les uns par rapport aux autres, le dispositif présente l'inconvénient de ne pas permettre un transfert qui soit efficace. En effet, avec de tels patchs prédécoupés, il se peut que l'utilisateur n'applique qu'en partie un des patchs prédécoupés.

On pourra également se référer au document EP 1 477 082 qui décrit un dispositif servant à appliquer des produits sur une mèche de cheveux. Mais il est également peu adapté pour l'application du patch sur la peau dans la mesure où il est agencé sous la forme d'une pince et n'est donc adapté que pour appliquer des produits sur deux faces d'une mèche.

La présente invention vise donc à remédier à ces inconvénients.

La présente invention a également pour but de prévoir un dispositif de transfert d'un patch ou film provenant d'une bande support qui soit particulièrement efficace, précis et simple à utiliser.

La présente invention a encore pour but un dispositif de transfert permettant d'obtenir une sensation de confort, lors de l'application du patch, et notamment lors de son application sur une partie du corps d'un utilisateur.

La présente invention a enfin pour but de prévoir un dispositif pour l'application de produits prédécoupés.

L'invention a donc pour objet un dispositif de transfert d'au moins un produit disposé sur une bande support. Ce dispositif est pourvu d'un boîtier, d'une bobine d'alimentation sur laquelle est enroulée la bande support du produit à transférer, et d'une bobine de réception de la bande support, les bobines d'alimentation et de réception étant montées à rotation sur le boîtier.

Selon une caractéristique générale du dispositif selon l'invention, la bande support comprend une pluralité de produits prédécoupés et le dispositif comprend en outre un organe d'actionnement mobile par rapport au boîtier et apte à entraîner en rotation l'une desdites bobines, de manière à permettre un déroulement d'une longueur prédéterminée de la bande support.

Avec un tel dispositif, il devient dès lors possible d'obtenir une application ou un transfert du ou des produits de la bande support sur une surface d'application de manière particulièrement satisfaisante et agréable pour un utilisateur du dispositif.

En effet, l'actionnement de l'organe mobile permet d'entraîner en rotation la bobine d'alimentation directement, ou indirectement via la bobine de réception, pour assurer un déroulement de la bande support.

Dans ces conditions, en vue de l'application du ou des produits, il n'est donc plus nécessaire d'exercer une pression importante sur la partie du corps à traiter afin d'obtenir la rotation de la bobine d'alimentation et le transfert du ou des produits.

En outre, la configuration de l'organe d'actionnement permet d'obtenir un déroulement de la bande support suivant une longueur prédéterminée, ce qui est particulièrement avantageux pour une bande support comprenant une pluralité de produits prédécoupés. En effet, l'organe d'actionnement est configuré de manière à obtenir l'avancement de la bande support d'une longueur égale au pas séparant deux produits consécutifs. On évite ainsi d'appliquer qu'en partie un des produits prédécoupés.

Dans un mode de réalisation préféré, l'organe d'actionnement est mobile à rotation par rapport au boîtier.

Préférentiellement, l'organe d'actionnement comprend une poignée d'avance et un levier de transmission du mouvement de la poignée à l'une desdites bobines.

Selon une autre caractéristique de l'invention, les bobines d'alimentation et de réception sont pourvues de roues dentées respectives coopérant l'une avec l'autre.

Dans un mode de réalisation préféré, le dispositif comprend en outre un organe de séparation du produit et de la bande support ménagé sur le boîtier et autour duquel la bande support forme une boucle.

Il peut en outre comprendre un patin d'application du produit disposé sur le boîtier, ledit patin d'application étant distinct de l'organe de séparation.

Une telle disposition permet d'obtenir un transfert particulièrement ergonomique du produit. En effet, l'utilisation d'un patin d'application qui soit spécifiquement dédié à l'application du patch sur la partie du corps à traiter permet d'accroître l'agrément de confort ressenti par un utilisateur du dispositif.

Avantageusement, le patin d'application est amovible. Il peut être réalisé en matière métallique.

Dans un mode de réalisation, le dispositif comprend en outre une cassette amovible à l'intérieur de laquelle sont disposées les bobines d'alimentation et de réception. La cassette peut comprendre un opercule amovible recouvrant un orifice de sortie pour la bande support.

Dans un mode de réalisation, le dispositif comprend une roue à rochet montée sur ladite cassette et apte à coopérer avec l'organe d'actionnement mobile pour l'entraînement en rotation de la bobine d'alimentation. En variante, la roue à rochet peut être montée sur le boîtier.

Avantageusement, le dispositif comprend en outre un organe élastique de rappel disposé entre le boîtier et l'organe d'actionnement mobile. De préférence, le dispositif est portatif.

Dans un mode de réalisation, les produits prédécoupés de la bande support sont espacés les uns par rapport aux autres. Avantageusement, lesdits produits prédécoupés sont des patchs.

La présente invention sera mieux comprise à la lecture de la description détaillée d'un mode de réalisation pris à titre d'exemple nullement limitatif et illustré par les dessins annexés, sur lesquels :
- la figure 1 est une vue en coupe en élévation d'un dispositif de transfert selon l'invention ;
- les figures 2 et 3 sont respectivement des vues en coupe et en perspective d'une cassette interchangeable du dispositif de la figure 1 ; et
- la figure 4 est une vue en perspective de la cassette interchangeable, à l'état ouvert.

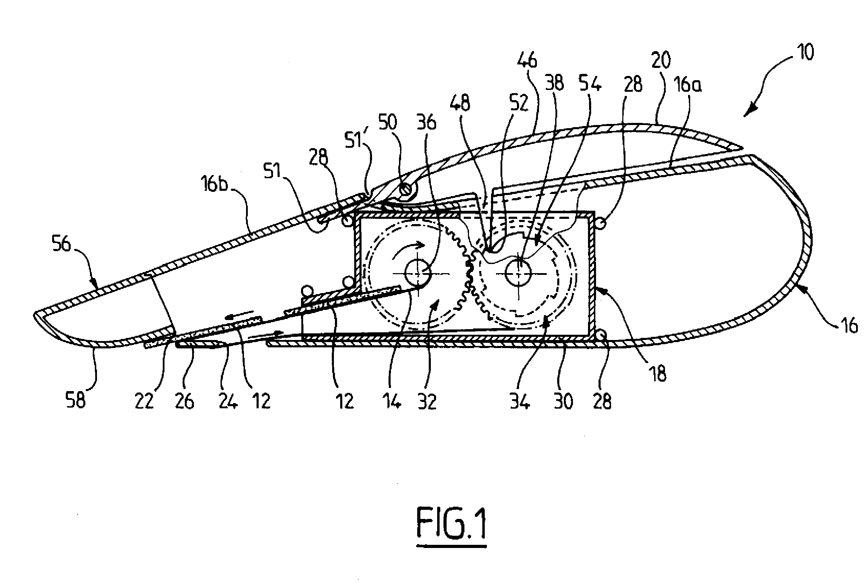

Sur la figure 1, on a représenté un dispositif de transfert, désigné par la référence numérique générale 10. I1 est destiné à être utilisé pour le transfert ou l'application sur l'épiderme d'une pluralité de patchs 12 contenant un produit cosmétique ou de soin disposés sur une bande support 14 et espacés les uns par rapport aux autres.

Toutefois, on conçoit aisément que le dispositif 10 peut être également utilisé pour l'application ou le transfert d'autres types de produits, ou encore être utilisé avec un film continu disposé sur la bande support.

Le dispositif 10 comprend un boîtier 16 à l'intérieur duquel est disposée une cassette 18 comprenant la bande support 14 et un organe d'actionnement mobile 20 apte à permettre un déroulement prédéterminé de ladite bande support 14 à l'extérieur de la cassette 18.

Le boîtier 16, de forme générale allongée, présente en section droite un profil sensiblement rectangulaire. Il comprend une base 16a conformée pour permettre le montage de la cassette 18 et pour définir, à une extrémité, une portion arrondie permettant à un utilisateur de maintenir le boîtier 16 contre la paume de sa main.

La base 16a est prolongée, du coté opposé à ladite portion arrondie, par une portion d'extrémité 16b présentant une forme générale en biseau. Cette portion d'extrémité 16b est pourvue à son extrémité libre d'un orifice de sortie 22. La base 16a et la portion d'extrémité 16b sont réalisées d'un seul tenant. Mais on pourrait également prévoir de réaliser la base 16a et l'extrémité 16b sous la forme de deux pièces distinctes fixées l'une sur l'autre, par exemple par encliquetage ou par tout autre moyen approprié.

Au voisinage dudit orifice de sortie 22, la portion d'extrémité 16b comprend également une ouverture 24 ménagée sur une face inférieure du boîtier 16. En d'autres termes, l'orifice de sortie 22 et l'ouverture 24 sont disposés au niveau d'une partie distale de la portion d'extrémité 16b du boîtier 16. L'ouverture 24 et l'orifice de sortie 22 laissent subsister entre eux une bande de matière formant organe de séparation 26 des patchs 12 de la bande support 14.

En effet, comme cela sera décrit en détail par la suite, lors de l'utilisation du dispositif 10, la bande support 14 est amenée à l'extérieur du dispositif à travers l'orifice de sortie 22 puis revient vers l'intérieur via l'ouverture 24 en formant une boucle autour de l'organe de séparation 26. L'organe de séparation 26 permet le détachement des patchs 12 de la bande support 14 en vue du transfert desdits patchs.

Le boîtier 16 est avantageusement réalisé à partir d'une matière synthétique moulée, par exemple du polypropylène. Comme cela est connu en soi, le boîtier 16 est avantageusement réalisé en deux parties (une seule partie étant visible sur la figure) s'emboîtant l'une dans à l'autre pour permettre l'insertion de la cassette 18 . En variante, il est également envisageable de prévoir un boîtier 16 en deux parties articulées l'une relativement à l'autre par l'intermédiaire d'une charnière.

De manière à permettre le positionnement de la cassette 18 à l'intérieur du boîtier 16, celui-ci comprend une pluralité de pions 28 de centrage venus de matière avec la partie correspondante dudit boîtier 16.

En ce qui concerne la cassette 18, celle-ci comprend un corps 30, de forme générale rectangulaire, à l'intérieur duquel sont montées une bobine d'alimentation 32 de la bande support 14 et une bobine de réception 34 de ladite bande support 14, une fois que les patchs 12 ont été transférés. La bobine d'alimentation 32 est montée libre à rotation sur un axe de support 36 du boîtier 16, tandis que la bobine de réception 34 est solidaire en rotation d'un axe support 38 du boîtier.

De manière classique, chacune des bobines 32, 34 est pourvue d'une portion annulaire d'enroulement de la bande support 14 et d'une roue dentée pour obtenir une coopération par engrènement avec l'autre bobine en vue du déroulement de la bande support 14.

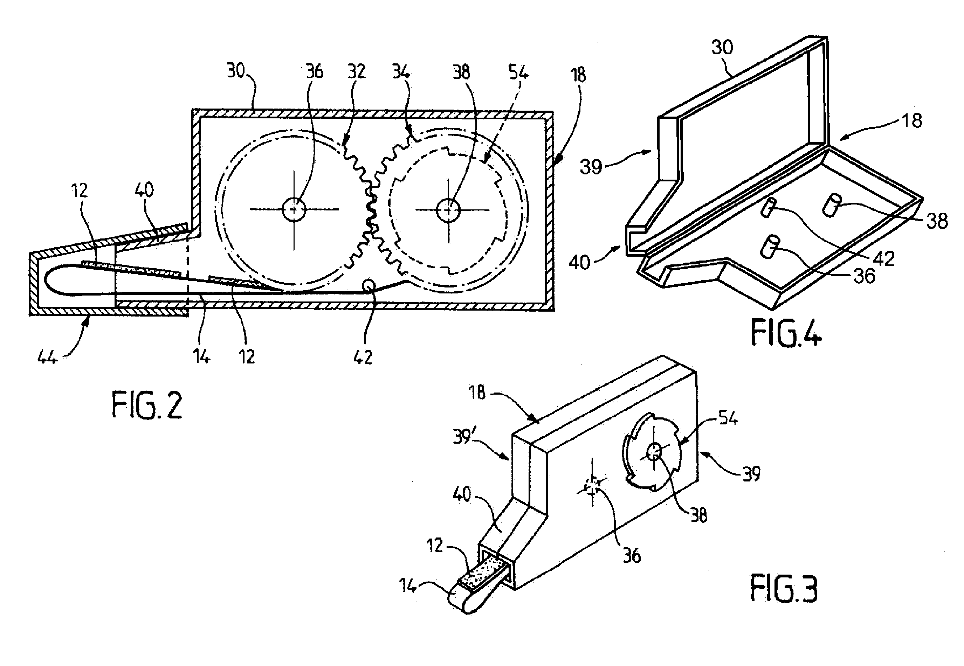

Comme illustré plus visiblement sur les figures 2 et 3, la cassette 18 est prolongée latéralement à une extrémité, par un col 40 ouvert à son extrémité libre de manière à permettre la sortie de la bande support 14. A cet égard, la cassette 18 comprend encore un pion de guidage 42 de la bande support 14 situé entre la bobine d'alimentation 32 et la bobine de réception 34. Avantageusement, la cassette 18 est réalisée en matière synthétique moulée, et par exemple transparente à la lumière du jour ou translucide. Elle peut toutefois être réalisée en matériau opaque à la lumière quand les patchs contiennent une composition sensible à la lumière.

On notera, comme illustré à la figure 4, que la cassette 18 est réalisée en deux parties 39 et 39' permettant l'introduction des bobines dans la cassette.

Dans l'exemple de réalisation illustré, les deux parties 39 et 39' sont articulées l'une sur l'autre par une charnière de liaison formée par l'un des côtés longitudinaux du corps de la cassette. Mais l'on pourrait également prévoir l'articulation des deux parties 39 et 39' au moyen d'une charnière formée par un côté transversal.

Dans l'exemple de réalisation illustré, les deux parties 39 et 39' sont venues de moulage. On pourrait également, en variante, réaliser ces parties sous la forme de deux pièces séparées venant se fixer l'une sur l'autre, par exemple par encliquetage, ou en utilisant tout autre moyen de fixation approprié.

Afin de parfaire l'assemblage et rendre l'assemblage étanche, on peut également prévoir de doter l'une des parties 39 ou 39' d'une lèvre d'étanchéité prévue sur son bord périphérique libre et destinée à coopérer avec le bord correspondant de l'autre partie ou avec une lèvre correspondante prévue sur cette autre partie afin de permettre de conserver les patchs à l'abri de l'air ambiant.

On notera enfin que, par exemple, dans le mode de réalisation illustré à la figure 4, les axes de support 36 et 38 et le pion de guidage 42 sont portés par l'une des parties 39 ou 39' du corps 40.

Avant l'introduction de la cassette 18 à l'intérieur du boîtier 16, celle-ci comprend également un opercule 44 amovible recouvrant l'ouverture du col 40 de manière à garantir une bonne étanchéité à l'intérieur de la cassette 18, pour notamment éviter l'intrusion d'éléments polluants susceptibles de se déposer au niveau de la bande support 14 et des patchs 12, ou encore d'éviter la détérioration du produit par évaporation.

L'opercule 44 comprend avantageusement une portion adhésive et une languette (non représentées) pour garantir son maintien sur le col 34 et faciliter son arrachement, respectivement.

L'opercule 44, de forme générale en C, est conformé de manière à recouvrir l'ouverture du col 40 tout en permettant à la bande support 14 d'être en saillie par rapport audit col. Ainsi, après l'arrachement de l'opercule 44, une extrémité en U de la bande support 14 peut être aisément tirée afin quelle forme une boucle autour de l'organe de séparation 24.

De manière à permettre le déroulement de la bande support 14 de la bobine d'alimentation 32, l'organe d'actionnement 20 (figure 1), réalisé en une seule pièce, comprend une poignée d'avance 46 montée à rotation sur le boîtier 16, et un levier de transmission 48 du mouvement de ladite poignée à la bobine de réception 34.

La poignée d'avance 46, de forme générale allongée, est articulée à rotation relativement au boîtier 16, via un axe de support 50 monté au niveau d'un évidement de la base 16a prévu à cet égard. La poignée d'avance 46 est configurée de manière à s'étendre au voisinage d'une partie supérieure de la base 16 sensiblement à partir de la portion d'extrémité 16b jusqu'à proximité de la portion arrondie de la base 16a. En outre, la poignée d'avance 46 est conformée afin qu'une partie supérieure présente une forme générale arrondie de manière à être ergonomique lorsque l'utilisateur amène son pouce ou son index contre cette poignée 46.

Elle est dotée d'une languette 51 qui prolonge la poignée 46 vers la portion d'extrémité 16b du boîtier 16.

Comme le montre la figure 1, cette languette s'insère à travers une fente transversale 51' prévue dans le boîtier pour venir en appui contre la surface interne de la paroi constitutive de la portion d'extrémité du boîtier. Cette languette 51 est élastiquement déformable et constitue ainsi un élément élastique sollicitant la poignée en position de repos relâchée. Mais on pourrait également réaliser cet organe de rappel élastique sous la forme d'un ressort prévu par exemple entre la poignée d'avance 46 et une surface d'appui correspondante pratiquée à cet effet dans le boîtier 16.

En ce qui concerne le levier de transmission 48, celui-ci se raccorde sur une partie inférieure de la poignée d'avance 46 en s'étendant sensiblement verticalement vers le bas, sous la forme d'un bras. Le levier de transmission 48 s'étend à l'intérieur du boîtier 16 à travers un orifice (non représenté) prévu à cet effet. I1 comprend à son extrémité libre une portion d'appui 52 en forme d'arc de cercle.

Cette dernière est en contact avec une roue 54 à rochet rapportée sur la cassette 18 en étant disposée au voisinage d'une face latérale de ladite cassette. Un jeu de fonctionnement est ménagé entre la roue 54 à rochet et la cassette 18 afin de limiter un éventuel contact de friction entre ces éléments. La roue 54 à rochet est liée à rotation avec la bobine de réception 34.

Dans ces conditions, lorsqu'un utilisateur appuie sur la poignée d'avance 46 de l'organe d'actionnement 20, elle se déplace en rotation autour de l'axe 50 et se rapproche du boîtier 16 jusqu'à venir en butée avec celui-ci. Ceci provoque également le déplacement selon une trajectoire circulaire autour de l'axe 50 du levier de transmission 48, dans le sens horaire sur la figure 1.

Ce faisant, la roue 54 à rochet et la bobine de réception 34 sont entraînées en rotation, dans le sens anti-horaire. Ainsi, la bobine d'alimentation 32 est également entraînée en rotation, dans le sens horaire, par engrènement avec ladite bobine de réception 34. On obtient ainsi le déroulement de la bande support 14.

Comme indiqué précédemment, afin de permettre le retour de la poignée d'avance 46 de l'organe d'actionnement 20 dans sa position initiale, et de pouvoir obtenir un nouveau déroulement de la bande support 14, le dispositif 10 comprend encore un organe de rappel élastique constitué par la languette 51. Lors du retour de la poignée d'avance 46 dans sa position initiale, le levier de transmission 48 n'exerce aucun effort au niveau de la roue 54 à rochet, ce qui limite sensiblement un éventuel déroulement intempestif de la bande support 14.

En d'autres termes, l'organe d'actionnement 20 est articulé à rotation relativement au boîtier 16, autour de l'axe 50, entre deux positions extrêmes de manière à permettre le déroulement de la bande support 14 d'une longueur prédéterminée qui correspond à l'espacement entre deux patchs 12 consécutifs. Comme on le conçoit aisément, la longueur de déroulement de la bande support 14, lors d'un actionnement de la poignée d'avance 46, est fonction de l'amplitude d'oscillation de l'organe d'actionnement 20 et de son dimensionnement.

En d'autres termes, le dispositif 10 est conçu pour obtenir, lors d'un actionnement de la poignée d'avance 46, un déroulement de la longueur de la bande support 14 permettant une application d'un seul patch 12.

Le dispositif 10 comprend en outre un embout 56, rapporté à l'extrémité libre de la portion d'extrémité 16b du boîtier 16, et pourvu d'un patin 58 d'application du patch 12.

L'embout 56 prolonge la portion d'extrémité 16b, du côté opposé à la base 16a. Il est monté de façon amovible sur la portion d'extrémité 16b, par exemple par encliquetage afin de faciliter son remplacement par un embout de forme différente qui soit adapté à la partie du corps à traiter. Le patin 58 d'application présente une forme générale recourbée. Il est avantageusement réalisé en matière métallique.

L'embout 56 et le patin 58 d'application sont conformés de manière à présenter une dimension transversale réduite par rapport à celle de la portion d'extrémité 16b afin de laisser libre en partie l'orifice de sortie 22 et à permettre l'amenée, à l'extérieur du dispositif 10, de la bande support 14, lors de l'actionnement de la poignée d'avance 46.

Comme mentionné précédemment, un tel actionnement provoque la rotation des bobines d'alimentation et de réception 32 et 34 en vue du déroulement de la bande support 14 pour l'application d'un unique patch 12, la bande support 14 formant une boucle tendue autour de l'organe de séparation 26.

Lorsqu'une extrémité avant d'un des patchs 12 est amené au niveau de l'orifice de sortie 22, l'organe de séparation 26 permet la désolidarisation progressive du patch 12 relativement à la bande support 12. Dès lors, le patch 12 continue à avancer vers le patin d'application 58 selon une même direction d'avance, tandis que la bande support 14 est dirigée, via l'ouverture 24, en direction de la bobine de réception 34. Ainsi, le patch 12 peut être appliqué sur la partie du corps à traiter.

De manière à faciliter cette désolidarisation, il est possible de prévoir des patchs 12 qui présentent chacun des extrémités avant et arrière découpée de manière à offrir une moindre adhérence sur la bande support 14 et faciliter ainsi leur détachement via l'organe de séparation 26, et leur application ou leur transfert par le patin 58. Les patchs peuvent par exemple être découpés de manière à présenter une forme en V à chacune de leur extrémité.

L'utilisation d'un patin 58 qui soit chacun spécifiquement dédié à l'application des patchs 12 sur la partie du corps à traiter permet d'accroître l'agrément de confort ressenti par un utilisateur du dispositif 10.

Dans le mode de réalisation illustré sur les figures, le dispositif 10 est conformé de manière à obtenir une application d'un seul patch 12, lors de l'actionnement de la poignée d'avance 46. Toutefois, on conçoit aisément qu'il est également envisageable de prévoir un dispositif permettant, lors d'un tel actionnement, d'appliquer plusieurs patchs 12.

Pour obtenir un déroulement de la bande support 14, le levier de transmission 48 coopère ici avec la bobine de réception 34. En variante, et en fonction du positionnement de la cassette 18 à l'intérieur du boîtier 16, il est toutefois également envisageable de prévoir un engagement du levier de transmission 48 directement avec la roue dentée de la bobine d'alimentation 32.

Dans un autre mode de réalisation, il pourrait également être envisageable de prévoir une roue 54 à rochet montée au niveau du boîtier 16.

## Revendications

1. Dispositif de transfert d'au moins un produit (12) disposé sur une bande support (14), le dispositif étant pourvu d'un boîtier (16), d'une bobine d'alimentation (32) sur laquelle est enroulée la bande support (14) du produit à transférer, et d'une bobine de réception (34) de la bande support, les bobines d'alimentation et de réception étant montées à rotation sur le boîtier, **caractérisé en ce que** la bande support (14) comprend une pluralité de produits prédécoupés, et **en ce qu'**il comprend en outre un organe d'actionnement (20) mobile par rapport au boîtier et apte à entraîner en rotation l'une desdites bobines de manière à permettre un déroulement d'une longueur prédéterminée de la bande support (14).

2. Dispositif selon la revendication 1, dans lequel l'organe d'actionnement (20) est mobile à rotation par rapport au boîtier.

3. Dispositif selon la revendication 1 ou 2, dans lequel l'organe d'actionnement (20) comprend une poignée d'avance (46) et un levier de transmission (48) du mouvement de la poignée d'avance à une desdites bobines.

4. Dispositif selon l'une quelconque des revendications 1 à 3, **caractérisé en ce que** les bobines d'alimentation et de réception (32, 34) sont pourvues de roues dentées respectives, coopérant l'une avec l'autre.

5. Dispositif selon l'une quelconque des revendications précédentes, comprenant en outre un organe de séparation (26) du produit et de la bande support ménagé sur le boîtier et autour duquel la bande support forme une boucle.

6. Dispositif selon la revendication 5, comprenant en outre un patin d'application (58) du produit disposé sur le boîtier, le patin d'application étant distinct de l'organe de séparation.

7. Dispositif selon la revendication 6, dans lequel le patin d'application est amovible.

8. Dispositif selon la revendication 6 ou 7, dans lequel le patin d'application est réalisé en matière métallique.

9. Dispositif selon l'une quelconque des revendications précédentes, comprenant en outre une cassette (18) amovible à l'intérieur de laquelle sont disposées les bobines d'alimentation et de réception (32, 34).

10. Dispositif selon la revendication 9, dans lequel la cassette comprend un opercule (38) recouvrant un orifice de sortie (22) pour la bande support.

11. Dispositif selon la revendication 8 ou 9, dans lequel la cassette (18) comprend une roue à rochet (54) apte à coopérer avec l'organe d'actionnement (20).

12. Dispositif selon l'une quelconque des revendications 1 à 10, comprenant en outre une roue à rochet (54) montée sur le boîtier et apte à coopérer avec l'organe d'actionnement (20).

13. Dispositif selon l'une quelconque des revendications précédentes, comprenant en outre un organe élastique de rappel disposé entre le boîtier et l'organe d'actionnement (20).

14. Dispositif selon l'une quelconque des revendications précédentes, dans lequel les produits prédécoupés de la bande support (14) sont espacés les uns par rapport aux autres.

15. Dispositif selon l'une quelconque des revendications précédentes, dans lequel les produits prédécoupés de la bande support (14) sont des patchs.

16. Utilisation d'un dispositif de transfert selon l'une quelconque des revendications précédentes 1 à 15 pour l'application de patchs contenant une composition cosmétique ou de soin.
